# EUROPEAN PATENT APPLICATION

(11) **EP 4 710 969 A1**
(43) Date of publication of application: **18.03.2026**
(21) Application number: 24306522.4
(22) Date of filing: 16.09.2024
(51) Int. Cl.: A61M 5/31

(54) **MEDICAL SYRINGE WITH REDUCED MATERIAL CONTENT**

(71) Applicant: Becton Dickinson France, 38800 Le Pont-de-Claix (FR)
(72) Inventor: LEIBBRAND, Alfred, 38640 Claix (FR); VACELET, Hélène, 38330 Montbonnot Saint Martin (FR)
(74) Representative: Germain Maureau

(57) **Abstract**

A syringe, comprising: a barrel defining a proximal end, a distal end, and an interior cavity, wherein the barrel defines: an outer diameter (D1), an inner diameter (D2), a wall thickness (T1), a height (H1) extending from the proximal end to the distal end, a form factor (FF) defined as H1/D1, a first volume (V1) of the interior cavity of at least 0.5 ml, a second volume (V2) of a material constituting the barrel, and a weight (W1) of the barrel, wherein: V2/V1 is between 0.5 and 1.6, W1/V1 is less than 4.2 g/ml, T1 is less than 1.0 mm, H1 is less than 62 mm, and FF is less than 6.0.

## Description

### FIELD OF THE INVENTION

The present disclosure is directed to medical containers, and in particular, to syringes having reduce material content and/or dimensions optimized for a pre-filled or predefined fluid volume.

### BACKGROUND OF THE INVENTION

Since their adoption in the medical field, glass pre-filled syringes have become a common drug administration device that are now governed by dedicated ISO standard to align all manufacturers on basic product specifications. These standardized specifications dictate the geometry and resulting volume of syringe barrels, resulting in virtually all modern day syringes conforming to a single geometry for a given container volume. Moreover, pharmaceutical companies support a growing trend towards accessory device platforms using the single, standardized syringe specifications for all their syringe needs in order to optimize procurement, storage, and operations with volume leverage. This can lead to systematically underfilling or underutilizing the total volumetric capacity of syringes for low dose applications (e.g. infant doses).

Recent efforts on sustainability and life cycle analysis indicates that the footprint of the glass used in pre-filled syringes is a significant waste contributor, including from its manufacture (energy-intensive melting of sand in furnaces), transformation (energy- intensive forming using gas burners), transportation (long distance shipping of heavy and fragile items) and finally, to its end-of-use (burial or incineration).

The present disclosure provides medical containers with reduced material content, optimized configurations, and sufficient strength or durability to reduce waste and improve sustainability.

### SUMMARY OF THE INVENTION

In accordance with one aspect, the present disclosure provides a syringe, comprising: a barrel defining a proximal end, a distal end, and an interior cavity, wherein the barrel defines: an outer diameter (D1), an inner diameter (D2), a wall thickness (T1), a height (H1) extending from the proximal end to the distal end, a form factor (FF) defined as H1/D1, a first volume (V1) of the interior cavity of at least 0.5 ml, a second volume (V2) of a material constituting the barrel, and a weight (W1) of the barrel, wherein: V2/V1 is between 0.5 and 1.6, W1/V1 is less than 4.2 g/ml, T1 is less than 1.0 mm, H1 is less than 62 mm, and FF is less than 6.0.

In some non-limiting embodiments or aspects, V1 may be 0.75 ml, and V2/V1 may be between 1.2 and 1.3.

In some non-limiting embodiments or aspects, W1/V1 may be less than 4.0 g/ml, and H1 may be less than 50 mm.

In some non-limiting embodiments or aspects, V1 may be 0.5 ml, and V2/V1 may be between 1.5 and 1.6.

In some non-limiting embodiments or aspects, H1 may be less than 40 mm, and FF may be less than 5.0.

In accordance with one aspect, the present disclosure provides a syringe, comprising: a barrel defining a proximal end, a distal end, and an interior cavity, wherein the barrel defines: an outer diameter (D1), an inner diameter (D2), a wall thickness (T1), a height (H1) extending from the proximal end to the distal end, a form factor (FF) defined as H1/D1, a first volume (V1) of the interior cavity of at least 1.0 ml, a second volume (V2) of a material constituting the barrel, and a weight (W1) of the barrel, wherein: V2/V1 is between 0.5 and 1.6, W1/V1 is less than 3.0 g/ml, T1 is less than 1.0 mm, H1 is less than 62 mm, and FF is less than 8.0.

In some non-limiting embodiments or aspects, V1 may be 1.25 ml, and V2/V1 may be between 1.0 and 1.2.

In accordance with one aspect, the present disclosure provides a syringe, comprising: a barrel defining a proximal end, a distal end, and an interior cavity, wherein the barrel defines: an outer diameter (D1), an inner diameter (D2), a wall thickness (T1), a height (H1) extending from the proximal end to the distal end, a form factor (FF) defined as H1/D1, a first volume (V1) of the interior cavity of at least 1.0 ml, a second volume (V2) of a material constituting the barrel, and a weight (W1) of the barrel, wherein: V2/V1 is between 0.5 and 1.6, W1/V1 is less than 4.2 g/ml, T1 is less than 1.1 mm, H1 is less than 50 mm, and FF is less than 4.0.

In some non-limiting embodiments or aspects, V2/V1 may be between 0.8 and 0.9, and W1/V1 may be less than 3.2 g/ml.

In some non-limiting embodiments or aspects, H1 may be less than 35 mm, and FF may be less than 3.5.

In some non-limiting embodiments or aspects, V1 may be 1.25 ml, and V2/V1 may be between 0.9 and 1.0.

In some non-limiting embodiments or aspects, W1/V1 may be less than 3.2 g/ml, and H1 may be less than 40 mm.

In some non-limiting embodiments or aspects, V1 may be 1.25 ml, and V2/V1 may be between 0.7 and 0.8.

In some non-limiting embodiments or aspects, W1/V1 may be less than 2.7 g/ml, T1 may be less than 0.9 mm, and H1 may be less than 40 mm.

In some non-limiting embodiments or aspects, W1/V1 may be less than 2.6 g/ml, T1 may be less than 0.9 mm, and H1 may be less than 45 mm.

In some non-limiting embodiments or aspects, V1 may be 1.25 ml, and V2/V1 may be between 0.55 and 0.65.

In some non-limiting embodiments or aspects, W1/V1 may be less than 2.3 g/ml, and T1 may be less than 0.7 mm.

In some non-limiting embodiments or aspects, H1 may be less than 40 mm, and FF may be less than 3.5.

In some non-limiting embodiments or aspects, V1 may be 3.0 ml, and V2/V1 may be between 0.7 and 0.8.

In some non-limiting embodiments or aspects, W1/V1 may be less than 2.5 g/ml, and FF may be less than 3.3.

Further examples of the present disclosure will now be described in the following numbered clauses.

Clause 1: A syringe, comprising: a barrel defining a proximal end, a distal end, and an interior cavity, wherein the barrel defines: an outer diameter (D1), an inner diameter (D2), a wall thickness (T1), a height (H1) extending from the proximal end to the distal end, a form factor (FF) defined as H1/D1, a first volume (V1) of the interior cavity of at least 0.5 ml, a second volume (V2) of a material constituting the barrel, and a weight (W1) of the barrel, wherein: V2/V1 is between 0.5 and 1.6, W1/V1 is less than 4.2 g/ml, T1 is less than 1.0 mm, H1 is less than 62 mm, and FF is less than 6.0.

Clause 2: The syringe of clause 1, wherein V1 is 0.75 ml, and V2/V1 is between 1.2 and 1.3.

Clause 3: The syringe of clause 1 or clause 2, wherein W1/V1 is less than 4.0 g/ml, and H1 is less than 50 mm.

Clause 4: The syringe of any of clauses 1-3, wherein V1 is 0.5 ml, and V2/V1 is between 1.5 and 1.6.

Clause 5: The syringe of any of clauses 1-4, The syringe of claim 4, wherein H1 is less than 40 mm, and FF is less than 5.0.

Clause 6: A syringe, comprising: a barrel defining a proximal end, a distal end, and an interior cavity, wherein the barrel defines: an outer diameter (D1), an inner diameter (D2), a wall thickness (T1), a height (H1) extending from the proximal end to the distal end, a form factor (FF) defined as H1/D1, a first volume (V1) of the interior cavity of at least 1.0 ml, a second volume (V2) of a material constituting the barrel, and a weight (W1) of the barrel, wherein: V2/V1 is between 0.5 and 1.6, W1/V1 is less than 3.0 g/ml, T1 is less than 1.0 mm, H1 is less than 62 mm, and FF is less than 8.0.

Clause 7: The syringe of clause 6, wherein V1 is 1.25 ml, and V2/V1 is between 1.0 and 1.2.

Clause 8: A syringe, comprising: a barrel defining a proximal end, a distal end, and an interior cavity, wherein the barrel defines: an outer diameter (D1), an inner diameter (D2), a wall thickness (T1), a height (H1) extending from the proximal end to the distal end, a form factor (FF) defined as H1/D1, a first volume (V1) of the interior cavity of at least 1.0 ml, a second volume (V2) of a material constituting the barrel, and a weight (W1) of the barrel, wherein: V2/V1 is between 0.5 and 1.6, W1/V1 is less than 4.2 g/ml, T1 is less than 1.1 mm, H1 is less than 50 mm, and FF is less than 4.0.

Clause 9: The syringe of clause 8, wherein V2/V1 is between 0.8 and 0.9, and W1/V1 is less than 3.2 g/ml.

Clause 10: The syringe of clause 8 or clause 9, wherein H1 is less than 35 mm, and FF is less than 3.5.

Clause 11: The syringe of any of clauses 8-10, wherein V1 is 1.25 ml, and V2/V1 is between 0.9 and 1.0.

Clause 12: The syringe of any of clauses 8-11, wherein W1/V1 is less than 3.2 g/ml, and H1 is less than 40 mm.

Clause 13: The syringe of any of clauses 8-12, wherein V1 is 1.25 ml, and V2/V1 is between 0.7 and 0.8.

Clause 14: The syringe of any of clauses 8-13, wherein W1/V1 is less than 2.7 g/ml, T1 is less than 0.9 mm, and H1 is less than 40 mm.

Clause 15: The syringe of any of clauses 8-14, wherein W1/V1 is less than 2.6 g/ml, T1 is less than 0.9 mm, and H1 is less than 45 mm.

Clause 16: The syringe of any of clauses 8-15, wherein V1 is 1.25 ml, and V2/V1 is between 0.55 and 0.65.

Clause 17: The syringe of any of clauses 8-16, wherein W1/V1 is less than 2.3 g/ml, and T1 is less than 0.7 mm.

Clause 18: The syringe of any of clauses 8-17, wherein H1 is less than 40 mm, and FF is less than 3.5.

Clause 19: The syringe of any of clauses 8-18, wherein V1 is 3.0 ml, and V2/V1 is between 0.7 and 0.8.

Clause 20: The syringe of any of clauses 8-19, wherein W1/V1 is less than 2.5 g/ml, and FF is less than 3.3.

### BRIEF DESCRIPTION OF THE DRAWINGS

The above-mentioned and other features and advantages of this disclosure, and the manner of attaining them, will become more apparent and the disclosure itself will be better understood by reference to the following descriptions of embodiments of the disclosure taken in conjunction with the accompanying drawings, wherein:

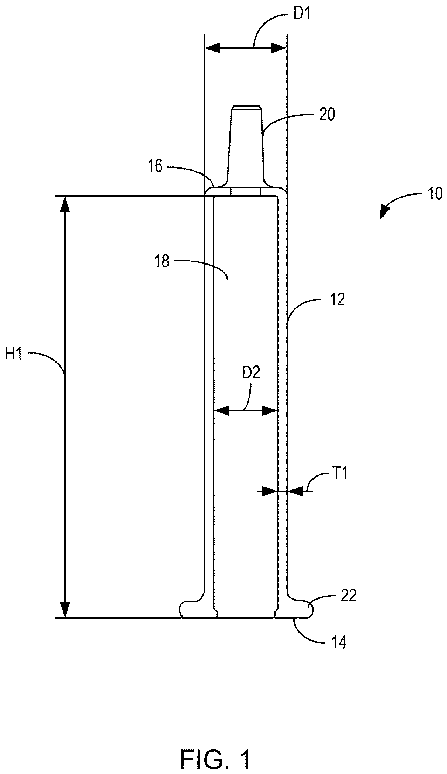

Fig. 1 is a side view of an example of a syringe constructed in accordance with the principles of the present invention.

### DESCRIPTION OF THE INVENTION

The following description is provided to enable those skilled in the art to make and use the described embodiments contemplated for carrying out the invention. Various modifications, equivalents, variations, and alternatives, however, will remain readily apparent to those skilled in the art. Any and all such modifications, variations, equivalents, and alternatives are intended to fall within the spirit and scope of the present invention.

For purposes of the description hereinafter, the terms "upper", "lower", "right", "left", "vertical", "horizontal", "top", "bottom", "lateral", "longitudinal", and derivatives thereof shall relate to the concept as it is oriented in the drawing figures. However, it is to be understood that the concept may assume various alternative variations, except where expressly specified to the contrary. It is also to be understood that the specific devices illustrated in the attached drawings, and described in the following specification, are simply exemplary embodiments of the concept. Hence, specific dimensions and other physical characteristics related to the embodiments disclosed herein are not to be considered as limiting.

The word "comprising" and "comprises", and the like, does not exclude the presence of elements or steps other than those listed in any claim or the specification as a whole. In the present specification, "comprises" means "includes" and "comprising" means "including."

As used herein, "at least one of" is synonymous with "one or more of." For example, the phrase "at least one of A, B, or C" means any one of A, B, or C, or any combination of any two or more of A, B, or C. For example, "at least one of A, B, and C" includes A alone; or B alone; or C alone; or A and B; or A and C; or B and C; or all of A, B, and C.

The term "at least" is synonymous with "greater than or equal to." The terms "first", "second", and the like are not intended to refer to any particular order or chronology, but refer to different conditions, properties, or elements. As used herein, the singular form of "a", "an", and "the" include plural referents unless the context clearly dictates otherwise.

All numbers used in the specification and claims are to be understood as being modified in all instances by the term "about". By "about" is meant within plus or minus twenty-five percent of the stated value. However, this should not be considered as limiting to any analysis of the values under the doctrine of equivalents.

Unless otherwise indicated, all ranges or ratios disclosed herein are to be understood to encompass the beginning and ending values and any and all subranges or subratios subsumed therein. For example, a stated range or ratio of "1 to 10" should be considered to include any and all subranges or subratios between (and inclusive of) the minimum value of 1 and the maximum value of 10; that is, all subranges or subratios beginning with a minimum value of 1 or more and ending with a maximum value of 10 or less. The ranges and/or ratios disclosed herein represent the average values over the specified range and/or ratio.

Reference is now made to Fig. 1, which shows a syringe, generally indicated as 10. The syringe 10 may include or define a barrel 12 having a proximal end 14, a distal end 16, and an interior cavity 18. The syringe 10 may include or define a distal tip 20 extending from the distal end of the barrel 12 for dispensing or dispersing a fluid from the cavity 18. The syringe 10 may include or define an opening at the proximal end 14 configured to receive a stopper and/or plunger (not shown), and may include or define a flange 22 at the proximal end circumscribing or otherwise extending around a perimeter of the proximal end 24.

The barrel 12 may define an outer diameter D1, an inner diameter D2 (e.g., the diameter of the interior cavity 18), and a height H1 of the interior cavity 18 extending from the proximal end 14 to the distal end 16. The barrel 12 may include or define a sidewall thickness T1, which can be calculated according to the equation T1 = (D1-D2)/2. The syringe 10 may include or define a form factor FF calculated according to the equation FF = H1/D1. The volume-to-surface ratio resulting from the form factor affects the storage / conservation of a liquid due to interactions between the contents of a container with the border/surfaces of the container.

The interior cavity 18 of the barrel 12 may define a volume V1, and the material (e.g., glass or otherwise) comprising the barrel 12 may define a volume V2. The material (e.g., glass or otherwise) comprising the barrel 12 may also define a weight W1.

The syringe 10 may have optimized, improved dimensions and configurations to reduce the raw materials necessary for manufacturing and other benefits disclosed herein, while accommodating a desired dosage or fluid volume therein. Such optimized, improved dimensions and configurations can also preserve or maintain the structural integrity or resistance to fracture similar to existing standardized syringes.

In one non-limiting example, the syringe 10 may have an inner volume capacity V1 of at least 0.5 ml, and may have a ratio of the volume V2 to the volume V1 between 0.5 and 1.6. The syringe may have a ratio of the weight W1 to the volume V1 equal to or less than 4.2 g/ml. The wall thickness T1 may be equal to or less than 1.0 mm. The height H1 may be equal to or less than 62 mm. The form factor FF may be equal to or less than 6.0.

In one non-limiting example, the syringe 10 may have an inner volume capacity V1 of at least 1 ml, and may have a ratio of the volume V2 to the volume V1 between 0.5 and 1.6. The syringe may have a ratio of the weight W1 to the volume V1 equal to or less than 3.0 g/ml. The wall thickness T1 may be equal to or less than 1.0 mm. The height H1 may be equal to or less than 62 mm. The form factor FF may be equal to or less than 8.0.

In one non-limiting example, the syringe 10 may have an inner volume capacity V1 of at least 1 ml, and may have a ratio of the volume V2 to the volume V1 between 0.5 and 1.6. The syringe may have a ratio of the weight W1 to the volume V1 equal to or less than 4.2 g/ml. The wall thickness T1 may be equal to or less than 1.1 mm. The height H1 may be equal to or less than 50 mm. The form factor FF may be equal to or less than 4.0.

In one non-limiting example, the syringe 10 may have an inner volume capacity V1 of approximately 1.25 ml, and may have a ratio of the volume V2 to the volume V1 between 1.0 and 1.2. The syringe may have a ratio of the weight W1 to the volume V1 equal to or less than 3.0 g/ml. The wall thickness T1 may be equal to or less than 1.0 mm. The height H1 may be equal to or less than 65 mm. The form factor FF may be equal to or less than 8.0.

In one non-limiting example, the syringe 10 may have an inner volume capacity V1 of approximately 0.75 ml, and may have a ratio of the volume V2 to the volume V1 between 1.2 and 1.3. The syringe may have a ratio of the weight W1 to the volume V1 equal to or less than 4.0 g/ml. The wall thickness T1 may be equal to or less than 1.0 mm. The height H1 may be equal to or less than 50 mm. The form factor FF may be equal to or less than 6.0.

In one non-limiting example, the syringe 10 may have an inner volume capacity V1 of approximately 0.50 ml, and may have a ratio of the volume V2 to the volume V1 between 1.5 and 1.6. The syringe may have a ratio of the weight W1 to the volume V1 equal to or less than 5.1 g/ml. The wall thickness T1 may be equal to or less than 1.0 mm. The height H1 may be equal to or less than 40 mm. The form factor FF may be equal to or less than 5.0.

In one non-limiting example, the syringe 10 may have an inner volume capacity V1 of approximately 1.0 ml, and may have a ratio of the volume V2 to the volume V1 between 0.8 and 0.9. The syringe may have a ratio of the weight W1 to the volume V1 equal to or less than 3.2 g/ml. The wall thickness T1 may be equal to or less than 0.9 mm. The height H1 may be equal to or less than 35 mm. The form factor FF may be equal to or less than 3.5.

In one non-limiting example, the syringe 10 may have an inner volume capacity V1 of approximately 1.25 ml, and may have a ratio of the volume V2 to the volume V1 between 0.9 and 1.0. The syringe may have a ratio of the weight W1 to the volume V1 equal to or less than 3.2 g/ml. The wall thickness T1 may be equal to or less than 1.0 mm. The height H1 may be equal to or less than 40 mm. The form factor FF may be equal to or less than 4.0.

In one non-limiting example, the syringe 10 may have an inner volume capacity V1 of approximately 1.25 ml, and may have a ratio of the volume V2 to the volume V1 between 0.7 and 0.8. The syringe may have a ratio of the weight W1 to the volume V1 equal to or less than 2.7 g/ml. The wall thickness T1 may be equal to or less than 0.9 mm. The height H1 may be equal to or less than 40 mm. The form factor FF may be equal to or less than 4.0.

In one non-limiting example, the syringe 10 may have an inner volume capacity V1 of approximately 1.25 ml, and may have a ratio of the volume V2 to the volume V1 between 0.7 and 0.8. The syringe may have a ratio of the weight W1 to the volume V1 equal to or less than 2.6 g/ml. The wall thickness T1 may be equal to or less than 0.9 mm. The height H1 may be equal to or less than 45 mm. The form factor FF may be equal to or less than 4.0.

In one non-limiting example, the syringe 10 may have an inner volume capacity V1 of approximately 1.25 ml, and may have a ratio of the volume V2 to the volume V1 between 0.55 and 0.65. The syringe may have a ratio of the weight W1 to the volume V1 equal to or less than 2.3 g/ml. The wall thickness T1 may be equal to or less than 0.7 mm. The height H1 may be equal to or less than 40 mm. The form factor FF may be equal to or less than 3.5.

In one non-limiting example, the syringe 10 may have an inner volume capacity V1 of approximately 3.0 ml, and may have a ratio of the volume V2 to the volume V1 between 0.7 and 0.8. The syringe may have a ratio of the weight W1 to the volume V1 equal to or less than 2.5 g/ml. The wall thickness T1 may be equal to or less than 1.1 mm. The height H1 may be equal to or less than 50 mm. The form factor FF may be equal to or less than 3.3.

The syringe 10 may include a film or coating of silicone on a surface of the interior cavity 18 to facilitate stopper movement and/or sealing of a fluid within the cavity 18. The silicone may be applied or deposited onto the surface of the interior cavity 18 in a lesser amount compared to that of the standardized syringes, while maintaining the operational benefits and features of a standardized syringe and plunger. In one example, the deposited film or coating of silicone may have a volume between 20 ×10⁻⁶ ml and 30 ×10⁻⁶ ml (and/or a weight between 46 × 10⁻³ mg and 69 ×10⁻³ mg for a silicone having a density of approximately 2,330 mg/ml).

The improved, optimized syringes disclosed herein use less glass material per syringe compared to current standardized configurations, which significantly reduces the volumes of glass purchased, transported, manipulated, processed to provide such syringed. Accordingly, throughout the life of the syringe (e.g., from procuring the raw materials to ultimate disposal of a used syringe) leads to substantial savings in terms of cost, energy, handling. In addition, manufacturing syringes with simpler, improved geometries as disclosed herein reduces the need for excessive quality controls and resulting scrap of non-conforming syringes. This also leads to savings in terms of cost, energy, and waste.

Moreover, the improved, optimized syringes disclosed herein can yield reduced industrial packaging between syringe manufacturers and pharmaceutical entities that fill the syringes. Pallets of syringes can be denser, contain more product and yield less waste packaging material. Handling, shipping, warehousing per syringe is substantially reduced, adding to the savings in cost, energy and waste. This is especially true in cold storage, once the syringes are full.

The improved, optimized syringes disclosed herein also allow reduced dimensions of an accompanying drug administration device (e.g. an autoinjector, pump, or other device) containing the syringe. This compounded benefit further reduces the amount of materials necessary to build such devices, their packaging, and reduce the resulting requirements for transportation and warehousing, further adding to substantial savings in cost, energy and waste.

Even further, the improved, optimized syringes disclosed herein reduce the overall footprint of a dose from manufacture to patient to end-of-life. The syringes (and any associated delivery device) allow reduced shelf-packaging as well as reduced transportation, handling, distribution and waste management.

It will be appreciated by persons skilled in the art that the present disclosure is not limited to what has been particularly shown and described herein above. In addition, unless mention was made above to the contrary, it should be noted that all of the accompanying drawings are not to scale. Of note, the system components have been represented where appropriate by conventional symbols in the drawings, showing only those specific details that are pertinent to understanding the embodiments of the present disclosure so as not to obscure the disclosure with details that will be readily apparent to those of ordinary skill in the art having the benefit of the description herein. Moreover, while certain embodiments or figures described herein may illustrate features not expressly indicated on other figures or embodiments, it is understood that the features and components of the examples disclosed herein are not necessarily exclusive of each other and may be included in a variety of different combinations or configurations without departing from the scope and spirit of the disclosure. A variety of modifications and variations are possible in light of the above teachings without departing from the scope and spirit of the disclosure, which is limited only by the following claims.

## Claims

1. A syringe, comprising:
a barrel defining a proximal end, a distal end, and an interior cavity, wherein the barrel defines:
an outer diameter (D1),
an inner diameter (D2),
a wall thickness (T1),
a height (H1) extending from the proximal end to the distal end,
a form factor (FF) defined as H1/D1,
a first volume (V1) of the interior cavity of at least 0.5 ml,
a second volume (V2) of a material constituting the barrel, and
a weight (W1) of the barrel,
wherein:
V2/V1 is between 0.5 and 1.6,
W1/V1 is less than 4.2 g/ml,
T1 is less than 1.0 mm,
H1 is less than 62 mm, and
FF is less than 6.0.

2. The syringe of claim 1, wherein V1 is 0.75 ml, and V2/V1 is between 1.2 and 1.3.

3. The syringe of claim 2, wherein W1/V1 is less than 4.0 g/ml, and H1 is less than 50 mm.

4. The syringe of claim 1, wherein V1 is 0.5 ml, and V2/V1 is between 1.5 and 1.6.

5. The syringe of claim 4, wherein H1 is less than 40 mm, and FF is less than 5.0.

6. A syringe, comprising:
a barrel defining a proximal end, a distal end, and an interior cavity, wherein the barrel defines:
an outer diameter (D1),
an inner diameter (D2),
a wall thickness (T1),
a height (H1) extending from the proximal end to the distal end,
a form factor (FF) defined as H1/D1,
a first volume (V1) of the interior cavity of at least 1.0 ml,
a second volume (V2) of a material constituting the barrel, and
a weight (W1) of the barrel,
wherein:
V2/V1 is between 0.5 and 1.6,
W1/V1 is less than 3.0 g/ml,
T1 is less than 1.0 mm,
H1 is less than 62 mm, and
FF is less than 8.0.

7. The syringe of claim 6, wherein V1 is 1.25 ml, and V2/V1 is between 1.0 and 1.2.

8. A syringe, comprising:
a barrel defining a proximal end, a distal end, and an interior cavity, wherein the barrel defines:
an outer diameter (D1),
an inner diameter (D2),
a wall thickness (T1),
a height (H1) extending from the proximal end to the distal end,
a form factor (FF) defined as H1/D1,
a first volume (V1) of the interior cavity of at least 1.0 ml,
a second volume (V2) of a material constituting the barrel, and
a weight (W1) of the barrel,
wherein:
V2/V1 is between 0.5 and 1.6,
W1/V1 is less than 4.2 g/ml,
T1 is less than 1.1 mm,
H1 is less than 50 mm, and
FF is less than 4.0.

9. The syringe of claim 8, wherein V2/V1 is between 0.8 and 0.9, and W1/V1 is less than 3.2 g/ml.

10. The syringe of claim 9, wherein H1 is less than 35 mm, and FF is less than 3.5.

11. The syringe of claim 8, wherein V1 is 1.25 ml, and V2/V1 is between 0.9 and 1.0.

12. The syringe of claim 11, wherein W1/V1 is less than 3.2 g/ml, and H1 is less than 40 mm.

13. The syringe of claim 8, wherein V1 is 1.25 ml, and V2/V1 is between 0.7 and 0.8.

14. The syringe of claim 13, wherein W1/V1 is less than 2.7 g/ml, T1 is less than 0.9 mm, and H1 is less than 40 mm.

15. The syringe of claim 13, wherein W1/V1 is less than 2.6 g/ml, T1 is less than 0.9 mm, and H1 is less than 45 mm.

16. The syringe of claim 8, wherein V1 is 1.25 ml, and V2/V1 is between 0.55 and 0.65.

17. The syringe of claim 16, wherein W1/V1 is less than 2.3 g/ml, and T1 is less than 0.7 mm.

18. The syringe of claim 17, wherein H1 is less than 40 mm, and FF is less than 3.5.

19. The syringe of claim 8, wherein V1 is 3.0 ml, and V2/V1 is between 0.7 and 0.8.

20. The syringe of claim 19, wherein W1/V1 is less than 2.5 g/ml, and FF is less than 3.3.
